# EUROPÄISCHE PATENTSCHRIFT

(11) **EP 2 473 835 B1**
(45) Veröffentlichungstag und Bekanntmachung des Hinweises auf die Patenterteilung: **18.05.2016**
(21) Anmeldenummer: 10768858.2
(22) Anmeldetag: 01.09.2010
(51) Int. Cl.: G01N 21/35, G01N 21/3504, G01N 33/497, A61B 5/083

(54) **VERFAHREN ZUR AUFZEICHNUNG UND AUSWERTUNG VON STOFFWECHSELVORGÄNGEN**
METHOD FOR RECORDING AND EVALUATING METABOLIC PROCESSES
PROCÉDÉ D'ENREGISTREMENT ET D'ÉVALUATION DES PROCESSUS DU MÉTABOLISME

(30) Priorität: 01.09.2009 DE 102009039543
(43) Veröffentlichungstag der Anmeldung: 11.07.2012
(73) Patentinhaber: ABB AG, 68309 Mannheim (DE)
(72) Erfinder: FABINSKI, Walter, 65830 Kriftel (DE); FISCHER, Heinz, 04155 Leipzig (DE); HÖLSCHER, Uvo, 48565 Steinfurt (DE); KAPPLER, Jürgen, 60435 Frankfurt Main (DE); VOGT, Josef, 89075 Ulm (DE)
(74) Vertreter: Schmidt, Karl Michael
(86) Internationale Anmeldenummer: PCT/EP2010/005367
(87) Internationale Veröffentlichungsnummer: WO 2011/026613

(56) Entgegenhaltungen:
- EP-A1- 0 627 195
- WO-A1-00/61197
- WO-A1-01/47416
- WO-A1-90/04164
- WO-A1-2007/012818
- WO-A1-2009/082318
- WO-A2-2007/000145
- US-A- 5 231 591
- US-A- 5 510 269
- US-A1- 2001 021 815
- US-A1- 2003 216 660
- US-A1- 2006 149 159
- US-A1- 2007 073 182
- US-A1- 2009 131 810
- US-B1- 6 272 905
- US-B1- 6 402 697
- ADAMEK R J ET AL: "13C-METHACETIN BREATH TEST: ISOTOPE-SELECTIVE NONDISPERSIVE INFRARED SPECTROMETRY IN COMPARISON TO ISOTOPE RATIO MASS SPECTROMETRY IN VOLUNTEERS AND PATIENTS WITH LIVER CIRRHOSIS", ZEITSCHRIFT FUER GASTROENTEROLOGIE, THIEME MEDICAL PUBLISHERS, DE, Bd. 37, Nr. 12, 1. Dezember 1999 (1999-12-01), Seiten 1139-1143, XP009072852, ISSN: 0044-2771
- BRADEN ET AL: "Methods and functions: Breath tests", BAILLIERE'S BEST PRACTICE AND RESEARCH. CLINICAL GASTROENTEROLOGY, BAILLIERE TINDALL, LONDON, US, Bd. 23, Nr. 3, 1. Juni 2009 (2009-06-01), Seiten 337-352, XP026281050, ISSN: 1521-6918, DOI: DOI:10.1016/J.BPG.2009.02.014 [gefunden am 2009-06-06]

## Beschreibung

Die Erfindung betrifft ein Verfahren zur Aufzeichnung und Auswertung von Stoffwechselvorgängen mit Hilfe einer isotopenspezifischen Analyse, gemäß des Patentanspruches 1.

Aus dem Stand der Technik sind diverse Verfahren dieser Art bekannt. So aus der EP1111371, sowie der DE19538431. Weitere Varianten zeigt die WO01/47416A1. Verfahren und Vorrichtungen zur offline-Diagnose von Stoffwechselvorgängen mit Hilfe der isotopenspezifischen Analyse des ¹³C Wertes mittels NDIR Spektroskopie sind aus den besagten EP1111371 und DE19538431 bekannt. Ein prominentes Beispiel ist hier die Diagnose auf Helicobakter Pylori. Ein weiteres Verfahren arbeitet bei erhöhter Sauerstoffzugabe. Sauerstoff und CO2 beeinflussen die ¹³C Messung bei der NDIR-Technik. Ein Beispiel für die Korrektur dieses Einflusses ist in der genannten WO 01/47416A1 aufgeführt. Dabei wird der Sauerstoffeinfluss durch Verdünnung mit Stickstoff eliminiert. Bei den o. genannten Verfahren wird die Patientenprobe als Atemgas in Beuteln zum Messgerät gebracht. Die Diagnose arbeitet damit nach dem besagten offline Prinzip.

Aus der US 2001/021815 A1 ist ein Verfahren der gattungsgemäßen Art bekannt. Bei diesem Verfahren ergibt sich in dem Verlauf der 13CO2-Konzentration bei einem schwer erkrankten Patienten eine vielhöckriger Kurvenverlauf, der von einer unsteten Magenentleerung bewirkt wird. Dies wiederum führt dazu, dass die Online-Messung der Stoffwechselaktivitäten davon überlagert und somit verfälscht wird.

Gewünscht werden darüber hinaus online Anwendungen, bei denen eine hohe Zeitauflösung auch bei gleichzeitig erhöhter Sauerstoffkonzentration notwendig ist. Dies ist z. B. der Fall bei der Bestimmung der Magenentleerzeit. Bei einer parenteralen Behandlung soll der Zeitpunkt des Beginns und des Fortganges der Oxidation möglichst genau bestimmt werden, um die Umstellung auf enterale Ernährung möglichst unmittelbar nach Einsetzen der Darmaktivität zu ermöglichen.
Eine Probenahme mit Beuteln ist für diese Anwendung zu langsam. Der Zeitpunkt der Umstellung soll aus dem Beginn der Oxidation als auch der Anstieg sicher und verlässlich erkennbar sein und durch Grenzwerte festgelegt werden. Dazu werden Grenzwerte festgelegt, die bei Überschreiten bewertet und Aktionen auslösen. Zur Plausibilisierung sind Prüfschritte zur Beurteilung des Messwertes vorgesehen. Sie erfolgen durch Aufgabe eines Prüfgases an der Enrnahmestelle.

Die Erfindung richtet sich auf ein Verfahren, bei dem eine zeitnahe Analyse durchgeführt werden kann. Dabei wird insbesondere die bei schwerkranken Patienten auftretende Mehrhöckrigkeit der 13CO2-Freisetzungkurven berücksichtigt, um dennoch zuverlässige Messergebnisse zu erhalten und die Problematik der offline-Entnahme zu überwinden, um schneller erforderliche Maßnahmen einleiten zu können.

Weitere vorteilhafte Ausgestaltungen sind in den abhängigen Ansprüchen 2 bis 13 angegeben.
Kern der verfahrensgemäßen Erfindung ist, dass der ¹³C Atemgaswert des Probanten mittels der NDIR-Spektroskopie (Nicht-Dispersive-Infrarot-Spektroskopie) kontinuierlich, d.h. online, mit einer Periode ermittelt und gespeichert wird, die deutlich kürzer ist als die biologische Halbwertzeit von Veränderungen im Stoffwechsel oder Konzentrationen in den Pools.
In vorteilhafter Ausgestaltung ist vorgesehen, dass die Periode der regelmäßigen Online-Messwiederholung kleiner als 1 Minute ist.
Weiterhin ist vorteilhaft ausgestaltet, dass die ermittelten Werte gespeichert und mit vorgegebenen Grenzwerten verglichen und bewertet werden.
Weiterhin ist ausgestaltet, dass parallel hierzu die 12C02 -Freisetzung pro Zeiteinheit des Patienten erfasst wird.
Weiterhin ist ausgestaltet, dass parallel hierzu die Magenentleerung des Patienten erfasst wird. durch Ermittlung von vorgegebenen Grenzwerten.
Weiterhin ist ausgestaltet, dass zur Festlegung der Grenzwerte des Rauschens oder Messfehler für die angegebenen Messungen vorgegeben und mit empirischen Werten verglichen werden.
Weiterhin ist ausgestaltet, dass auch andere, zusätzliche Parameter zur Beschreibung des Allgemein-Zustandes erfasst werden, wie z.B. Alter, Gewicht, des Probanten, sowie Muster der vom Körper produzierten Atemgase wie etwa Keto-Körper.

Es ist vorgesehen, dass aus den genannten Messungen und erfassten Zustandsdaten über Rechenmodelle Magenentleerung und Aufnahme über den Darm getrennt ermittelt und erfasst werden, und dass für Magenentleerung und Darm-Aufnahme Indikatorvariablen generiert werden. D.h. die Indikator-Variablen können kinetisch bestimmte Parameter sein, wie etwa Prozentsatz des verabreichten Tracers, der zu einem bestimmten Zeitpunkt abgeatmet wurde, oder Zeitpunkt, bei dem die Hälfte der Menge, die dem Magen zugeführt wurde, in den Darm abgegeben wurde.
Weiterhin ist ausgestaltet, dass aus den abgeschätzten Messfehler ein Zuverlässigkeitsmass in Form eines Streubereiches für die Indikatorvariable erstellt wird, und dass für diese Indikatorvariablen und deren Streubereich Grenzwerte vorgegeben werden, anhand derer die Umstellung von parenteraler auf enterale Ernährung ggfs festgelegt bzw automatisch generiert wird.

Weiterhin ist ausgestaltet, dass wahlweise zuerst die einfachste Variante des Atemgastests, basierend alleine auf die 13CO2 Anreicherung durchgeführt wird, und dass für die besagten Indikatorvariablen eine maximale Streubreite festgelegt wird, bei deren Überschreitung der Test wiederholt oder partielle Zusatzmessungen wie Magenentleerung durchgeführt werden, um auf umfangreichere und robustere Auswerte-Modelle nur dann zurück zu greifen, wenn diese tatsächlich benötigt werden.

Weiterhin ist ausgestaltet, dass die Messung des CO2 Flows über einen Bypass mittels Kapillar- / Differenzdruckmethode vorgenommen wird.

Weiterhin ist ausgestaltet, dass Konzentrationsfehlmessungen durch Magenabsonderungen durch eine Salzmessung korrigiert werden.

Weiterhin ist ausgestaltet, dass Plausibilitätsprüfungen, insbesondere bei Auftreten von Änderungen des d13C-Wertes durchführbar sind, durch Aufgabe eines dem Probanten ähnlichen Ausexpirationsluft am Eingang der Probenahme, oder durch Aufgabe der Gasprobe am Eingang der Probenahme durch Überströmen des Meßgases mit Prüfgas.

Die Etablierung eines online Verfahrens unter variablen Sauerstoff-Konzentrationen im Atemgas alleine reicht nicht aus um ein Atemgas-Test für Intensiv - Patienten durchzusetzen.
Der Atemgas-Test zur Bestimmung der Magenentleerung verwendet im wesentlichen das zeitliche Profil der 13CO2-Anreicherung nach Verabreichung der markierten Testsubstanz. Aus dem Anstieg zu einem Maximum, gefolgt von einem exponentiellen "Abklingen" der Anreicherung lässt sich der Transport des Tracers von Magen in stoffwechsel-aktive Organe herleiten.

Die Analyse beruht auf folgenden Annahmen:
a) die Magenentleerung läuft kontinuierlich ab.
b) Der Transfer vom Darm in die Zirkulation und stoffwechsel-aktive Organe, exemplarisch im folgenden die Leber, läuft so schnell und ungestört ab, dass die Magenentleerung der eigentlich zeitlich limitierende Schritt für den Transport des Tracer zur Leber ist.
c) der Umbau des Tracers zu 13CO2 ("Oxidation") läuft immer mit gleicher Effizienz ab.
d) Das produzierte 13CO2 sammelt sich in einem Pool (zirkulierendes Blut) und wird von dort abgeatmet.

Bei schwerkranken Patienten kann jedoch die Magenentleerung zeitlich unterbrochen sein, so daß Annahme a) nicht mehr zutrifft und unter Umständen mehr-höckrige 13CO2 -Freisetzungskurven sichtbar werden. Außerdem kann neben dem Magen auch die Dünndarm Aktivität gestört sein, so daß Annahme b) nicht mehr zutrifft. Daher ist zu erwarten, daß für eine Reihe von schwer-kranken Patienten der Test entweder generell nicht anwendbar ist, oder unzuverlässige Ergebnisse liefert.
Deswegen sollen eine Reihe von Modifikationen und Erweiterungen des Tests vorgenommen werden, un sicherzustellen, dass auf jedem Fall brauchbare Ergebnisse erzielt werden. Um das Problem mehrhöckriger Freisetzungskurven zu umgehen, soll notfalls die kinetische Auswertung durch eine Bilanz-orientierte Auswertung ersetzt oder ergänzt werden.

Hierfür wird die absolute, kumulative Menge von 13CO2, die bis zu einem bestimmten Zeitpunkt abgeatmet wurde als Indikator für die Menge an Testsubstanz verwendet werden, die vom Magen in den Darm freigegeben wurden. Dafür muss neben der 13CO2 Anreicherung im Atemgas auch die zeitlich ausgelöste absolute Menge an abgeatmeten Gesamt-CO2 erfasst werden. Die entsprechenden technischen Massnahmen werden im Punkt "Erf.-gemäße Ausführung"aufgeführt.
Die Analyse der Atemgaswerte nach dem etablierten Schema setzt eine ungestörte Dünndarmaktivität-und Motilität vorraus, und reduziert Verwertungsprobleme auf Magenentleerungsprobleme. Ohne ausreichende Motilität gleicht der Darm jedoch einem gefüllten Schlauch, bei dem zwar in Nähe der Darmwand eine Resorption auftritt, Material in der Mitte des Darmlumens muss jedoch über passive Diffusion zur Darmwand gelangen, was zu zeitlichen Verzögerungen führen kann. Nur mit aktiver Durchmischung des Darminhaltes kann dieser passive Diffusionsprozess vermieden werden. Magenentleerung und Darm-absorption sollten daher getrennt erfasst werden.

Deswegen wird eine 13CO2 unabhängige Erfassung der Entleerung des Magens angestrebt.

Seine Bilanz wird durch Nahrungszufuhr, Weiterleitung in den Darm sowie Sekretion weitgehend bestimmt. Da die Nahrungszufuhr bekannt, die Weiterleitung in den Darm bestimmt werden soll, ist Sekretion eine Unbekannte. Sie kann durch Zugabe eines weiteren Tracers in die Nahrung bestimmt werden. Hierzu gibt man eine im Magen inerte, physiologisch unbedenkliche Substanz in fixer Konzentration der Nahrung bei. Wenn man diese Konzentration dann im Magen bestimmen kann, ergibt sich damit die Sekretion. Die Bestimmung kann durch kurzfristiges Abziehen einer kleinen Menge aus dem Magen und externer Diagnose erfolgen.

Auch ist es möglich, die Konzentration durch physikalische oder technische Verfahren im Magen durch einen Sensor zu bestimmen. Hier bieten sich Verfahren an, die spezifisch auf den weiteren Tracer reagieren. Besteht der Tracer zum Beispiel aus einem Farbstoff wäre die Messung über die Farbe möglich, bei einem elektrochemisch durch Leitfähigkeit oder Polarogramm zu bestimmenden Tracer käme die Methode zu Zuge. Besitzt ein Tracer aus Gasblasen deutlich andere physikalische Eigenschaften als das Sekret kann man über mechanische oder akustische oder weitere physikalische Methoden die verbliebene Tracerkonzentration bestimmen.

Mit der Information aus gemessener Magenentleerung, zeitlichen Profil der 13CO2 Freisetzung (als absolute Menge) sollte es möglich sein, Probleme in der Absorption im Darm von Magenentleerung Problemen zu trennen. Dies muß über eine kinetische Modellrechnung geschehen. Diese Modelle benötigen zusätzliche Informationen, z.B. die Menge an CO2, das im Blut vorübergehen gebunden ist, oder Anteil des Tracers, der in der Leber zu 13CO2 abgebaut wird.

Diese zusätzlichen Parameter hängen von Alter, Gewicht und Krankheitsbild eines Patienten ab. Um diesen zu charakterisieren wird die Meßeinrichtung erf.gemäss so erweitert, so dass weitere Messgrößen der Patienten durch Sensoren (zum Beispiel Temperatur, endtidaler Atem-CO2) oder durch Eingabevorrichtungen (für zum Beispiel Gewicht, Krankheitsbilder, laboranalytisch bestimmte Werte) erfasst werden. So können zum Beispiel aus der Atemmenge und der CO2-Konzentration qualitative Werte für die pool-Konzentrationen sowie die Stoffwechselmengen gewonnen werden können, um den genauen Punkt der Umstellung auf enterale Ernährung zu bestimmen. Zusätzlich kann ein weiteres Gerät integriert werden, das analog den Umsatz eines oder mehrerer weitere Isotope analysiert. Beispiele hierfür sind die Isotope von Wasser-, Stick- und Sauerstoff. Im Fall von stabilen Isotopen geht die Messung ohne radiologische Belastung von Patient und Umwelt einher.

Abschätzung der Magenentleerung über 13CO2 Isotope: Der Test ist für eine einfache Ausführung und Auswertung optimiert. Von Interesse ist das zeitliche Profil der 13CO2-Anreicherung im Atemgas nach Gabe einer markierten Testsubstanz. Dieses Profil zeigt in der Regel ein Maximum nach etwa 30-60 Minuten, gefolgt von einer Abklingkurve, die mit einer exponentiellen Funktion approximiert werden kann. Ist dieser Verlauf gegeben, dann kann aus ihm abgeschätzt werden, wie schnell sich der Magen entleert. Die Abschätzung beruht auf der Annahme, daß die Magenleerung der einzig zeitlich limitierende Schritt ist und damit auch die Kinetik bestimmt. Das Auswerte - Verfahren versagt, wenn die Magenentleerung zeitweise blockiert und diskontinuierlich wird. Auswerteverfahren für diesen Fall sind nicht etabliert. Es gibt keine Ansätze, Störungen im Darmbereich und Störungen in der Magen-Entleerungen getrennt zu erfassen.

NDIR-Technologie zur online-Erfassung der 13CO2 Anreicherung: Eine adäquate Routinediagnosetechnik durch schnelle online Messung der ¹³C Konzentration zum Einsatz in Intensivstationen für die Ermittlung der Magenentleerzeit , die die o. gestellten Forderungen erfüllt, ist bis heute nicht bekannt. Die bekannte Massenspektroskopie ist nicht online fähig und benötigt unter den Bedingungen der Intensivstation viel Platz, ist aufwendig in der Anschaffung und Unterhaltung und benötigt intensive Betreuung.

Erfassung der Menge an abgeatmeten CO2: Dies ist ein establiertes Verfahren für Isotopen-gestützten Stoffwechsel-Untersuchung. Hier wird aus dem Produkt von abgeatmeter Gesamt-CO2 Menge und 13CO2 Anreicherung die insgesamt abgeatmete 13CO2 Menge abgeschätzt und für Isotopen-Bilanz Rechnungen herangezogen. In der Regel wird die abgeatmeten CO2 Menge über ein gesondertes Gerät erfasst. Um den zusätzlichen apparativen Aufwand zu vermeiden verzichten Atemgas-Tests verzichten auf diese Messungen.

Erfassung der Magenentleerung: Gold-Standard zur Erfassung der Magenentleerung ist ein bildgebendes, szintigraphisches Verfahren, das in verschiedenen Zeitabständen die Rest-Menge eines Kontrast-Mittels im Magen bestimmt. Als einfache alternative kann der Nahrungsanteil des Mageninhaltes über den Brechungsindex bestimmt werden, da fetthaltige Nahrungslösungen ein anderen Brechungsindex haben als die klaren Sekretionslösungen des Magens. Bei Gesunden scheint die szintigrafisch bestimmte Magenentleerung der Abschätzung über den Atemgas-Test zu entsprechen. Beide Verfahren beruhen auf der Annahme, dass Störungen in der Entleerung repräsentativ für Störungen im Magen-Darm-Kanal sind.

Eine erfindungsgemäße Ausführung lässt sich wie folgt zusammen fassen:
1. NDIR-Technik: Die hier vorgeschlagene Verfahren und die Einrichtung überkommt die Probleme der MS-Technik und der offline Entnahme durch Einsatz der NDIR-Technik und ist darüber hinaus robust, kostengünstig und mit kleinen Abmessungen damit für den online Einsatz auf Intensivstationen geeignet.
2. Erfassung der CO2-Freisetzung durch NDIR-Messtechnik.
3. Erfassung der Magenentleerung: Hier bietet sich jedes Verfahren an, mit den der Mageninhalt markiert und gemessen werden kann. Wenn ein Farbstoff als Tracer zur Magenentleerung eingesetzt wird, könnte dessen Konzentration über photometrische Verfahren gemessen werden. Mit Mehrkanal SpektroPhotometrie sollten Spektren (und damit Konzentrationen) des Farbstoffes von Spektren der Sekretflüssigkeit oder Spektren der Nahrungslösung trennbar sein. Dies würde die Chance bieten, die Dynamik der Veränderung des Mageninhaltes genauer zu erfassen. In der Literatur vorgeschlagen wird die Messung des "Brechungs-Index" der Magenflüssigkeit, da fetthaltige Nahrungslösungen einen anderen Index haben als Magensekrete.
4. Plausibilitätstest.
   Insbesondere bei Auffälligkeiten in der d13C-Messung wird ein Plausibilitätstes durch Aufgabe eine Nullgases an der Entnahmestelle z.B. durch Überströmes des Meßgases durchgeführt und bewertet (s. Bild Anlage)
5. Modell-Rechnung zur Auswertung der 13CO2 und Magenentleerungsdaten. Gängige Modelle werden vor allem dadurch erweitert, dass eine Verzögerung in der Aufnahme des Tracers über den Darm berücksichtigt wird.
6. Charakterisierung des Patienten: Die N20 Produktion in der Lunge ist bei kritisch Kranken verändert. Ebenso die Produktion kurz-kettiger Aldehyde /Ketone wie Azeton. Deren Konzentrations-Muster könnte zur Charakterisierung des Patienten herangezogen werden und über Infra-Rot Spektroskopie gemessen werden. Vorteilhaft sind Analyseverfahren, die gasförmige Ausscheidungsprodukte messen. Im Fall von Stickstoff findet die Ausscheidung primär über den Urin statt, so dass hier eine chemisch-physikalische Umsetzung von der flüssigen in die gasförmige Phase zwischengeschaltet werden muss. Im Prinzip eignet sich das Lehrer-Luft Verfahren für alle Isotopenmessungen.

## Patentansprüche

1. Verfahren zur Aufzeichnung und Auswertung von Stoffwechselvorgängen durch eine Ermittlung der Magenentleerzeit oder durch eine Ermittlung der Interaktion von Medikamenten bei der Polymedikamentation über eine Ermittlung der Magenentleerzeit mit Hilfe der isotopenspezifischen Analyse, bei welchem die Magenentleerzeiten mittels des Quotienten der 13CO2- und 12CO2-Konzentration in der Probanden-Expirationsluft ermittelt werden, wobei der ¹³C Atemgaswert der Probanden mittels der Nicht-Dispersiven-Infrarot-Spektroskopie kontinuierlich, online und periodisch ermittelt und gespeichert wird, mit einer Periode, die deutlich kürzer ist als die biologische Halbwertzeit von Veränderungen im Stoffwechsel oder Konzentrationen in den Pools von zirkulierendem Blut, und wobei eine kinetische Auswertung der Atemgasewerte durch eine bilanzorientierte Auswertung ersetzt oder ergänzt wird, indem eine kumulative Menge an 13CO2, die bis zu einem bestimmten Zeitpunkt nach Verabreichung einer Testsubstanz abgeatmet wurde, als Indikator für die Menge an Testsubstanz verwendet wird, die vom Magen bis zu diesem Zeitpunkt in den Darm freigegeben wird, wobei zur Ermittlung der Magenentleerzeiten neben der 13CO2 Anreicherung im Atemgas auch die zeitlich ausgelöste absolute Menge an abgeatmetem Gesamt-CO2 bis zu diesem Zeitpunkt erfasst wird, und wobei aus den genannten ermittelten Magenentleerzeiten und weiteren erfassten Zustandsdaten, über Rechenmodelle, Magenentleerung und Aufnahme über den Darm getrennt ermittelt und erfasst werden und für Magenentleerung und Darm-Aufnahme Indikatorvariablen generiert werden.

2. Verfahren nach Anspruch 1,
**dadurch gekennzeichnet,**
**dass** die Periode der regelmäßigen Online-Messwiederholung kleiner als 1 Minute ist.

3. Verfahren nach Anspruch 1 oder 2,
**dadurch gekennzeichnet,**
**dass** die ermittelten Werte gespeichert und mit vorgegebenen Grenzwerten verglichen und bewertet werden.

4. Verfahren nach einem der vorhergehenden Ansprüche,
**dadurch gekennzeichnet,**
**dass** parallel hierzu die 12CO2 -Freisetzung pro Zeiteinheit des Patienten erfasst wird.

5. Verfahren nach einem der vorhergehenden Ansprüche,
**dadurch gekennzeichnet,**
**dass** parallel hierzu die Magenentleerung des Patienten erfasst wird durch Ermittlung von vorgegebenen Grenzwerten.

6. Verfahren nach einem der vorhergehenden Ansprüche,
**dadurch gekennzeichnet,**
**dass** zur Festlegung der Grenzwerte des Rauschens oder Messfehler für die angegebenen Messungen vorgegeben und mit empirischen Werten verglichen werden.

7. Verfahren nach einem der vorhergehenden Ansprüche,
**dadurch gekennzeichnet,**
**dass** auch andere, zusätzliche Parameter zur Beschreibung des Allgemein-Zustandes erfasst werden, wie z.B. Alter, Gewicht, des Probanten, sowie Muster der vom Körper produzierten Atemgase wie etwa Keto-Körper.

8. Verfahren nach einem der vorhergehenden Ansprüche,
**dadurch gekennzeichnet,**
**dass** aus den abgeschätzten Messfehler ein Zuverlässigkeitsmass in Form eines Streubereiches für die Indikatorvariable erstellt wird, und dass für diese Indikatorvariablen und deren Streubereich Grenzwerte vorgegeben werden, anhand derer die Umstellung von parenteraler auf enterale Ernährung ggfs festgelegt bzw automatisch generiert wird.

9. Verfahren nach einem der vorhergehenden Ansprüche,
**dadurch gekennzeichnet,**
**dass** wahlweise zuerst die einfachste Variante des Atemgastests, basierend alleine auf die 13CO2 Anreicherung durchgeführt wird, und dass für die besagten Indikatorvariablen eine maximale Streubreite festgelegt wird, bei deren Überschreitung der Test wiederholt oder partielle Zusatzmessungen wie Magenentleerung durchgeführt werden, um auf umfangreichere und robustere Auswerte-Modelle nur dann zurück zu greifen, wenn diese tatsächlich benötigt werden.

10. Verfahren nach einem der vorhergehenden Ansprüche,
**dadurch gekennzeichnet,**
**dass** die Messung des CO2 Flows über einen Bypass mittels Kapillar- / Differenzdruckmethode vorgenommen wird.

11. Verfahren nach einem der vorhergehenden Ansprüche,
**dadurch gekennzeichnet,**
**dass** Konzentrationsfehlmessungen durch Magenabsonderungen durch eine Salzmessung korrigiert werden.

12. Verfahren nach einem der vorhergehenden Ansprüche,
**dadurch gekennzeichnet,**
**dass** Plausibilitätsprüfungen, insbesondere bei Auftreten von Änderungen des d13C-Wertes durchführbar sind, durch Aufgabe eines dem Probanten ähnlichen Ausexpirationsluft am Eingang der Probenahme, oder durch Aufgabe der Gasprobe am Eingang der Probenahme durch Überströmen des Meßgases mit Prüfgas.

## Claims

1. Method for recording and evaluating metabolic processes by means of a determination of the gastric emptying time or by means of a determination of the interaction of medicaments in the polymedication via a determination of the gastric emptying time with the aid of isotope-specific analysis, in which method the gastric emptying times are determined by means of the quotient of the 13CO2 concentration and 12CO2 concentration in the test subject expiration air, wherein the ¹³C respiratory gas value of the test subject is determined and stored by means of non-dispersive infrared spectroscopy in a continuous, online and periodic manner, with a period which is distinctly shorter than the biological half-life of changes in metabolism or concentrations in the pools of circulating blood, and wherein a kinetic evaluation of the respiratory gas values is replaced or supplemented by a balance-oriented evaluation, by using a cumulative amount of 13CO2 which has been breathed out up to a particular time point after administration of a test substance as an indicator for the amount of test substance which is released into the intestine from the stomach up to said time point, wherein the gastric emptying times are determined by capturing not only the 13CO2 enrichment in the respiratory gas but also the time-triggered absolute amount of breathed-out total CO2 up to said time point, and wherein, from said determined gastric emptying times and further captured status data, gastric emptying and absorption across the intestine are, via computational models, determined and captured separately and indicator variables are generated for gastric emptying and intestinal absorption.

2. Method according to Claim 1,
**characterized in that**
the period of the regular online repeated measurement is smaller than 1 minute.

3. Method according to Claim 1 or 2,
**characterized in that**
the determined values are stored and compared and assessed with predefined limit values.

4. Method according to any of the preceding claims,
**characterized in that**
the 12CO2 release per unit time of the patient is captured in parallel thereto.

5. Method according to any of the preceding claims,
**characterized in that**
the gastric emptying of the patient is captured in parallel thereto by determination of predefined limit values.

6. Method according to any of the preceding claims,
**characterized in that**
for the definition of the limit values of the noise or measurement errors for the specified measurements are predefined and compared with empirical values.

7. Method according to any of the preceding claims,
**characterized in that**
other additional parameters for describing the general status are captured too, such as, for example, age, weight, of the test subject, and also samples of respiratory gases produced by the body, such as ketone bodies for instance.

8. Method according to any of the preceding claims,
**characterized in that**
a measure of reliability in the form of a scatter range for the indicator variables is created from the estimated measurement errors, and that limit values are predefined for said indicator variables and the scatter range thereof, on the basis of which limit values the changeover from parenteral to enteral nutrition is defined or automatically generated as the circumstances require.

9. Method according to any of the preceding claims,
**characterized in that**
the simplest variant of the respiratory gas test, based solely on the 13CO2 enrichment, is firstly carried out on the basis of choice, and that a maximum scatter range is defined for said indicator variables, the test being repeated or partial additional measurements such as gastric emptying being carried out if said maximum scatter range is exceeded, so as to only resort to more comprehensive and more robust evaluation models when said models are actually required.

10. Method according to any of the preceding claims,
**characterized in that**
the measurement of the CO2 flow across a bypass is performed by means of a capillary method/differential pressure method.

11. Method according to any of the preceding claims,
**characterized in that**
incorrect concentration measurements due to gastric secretions are corrected by a salt measurement.

12. Method according to any of the preceding claims,
**characterized in that**
it is possible to carry out plausibility tests, especially in the case of occurrence of changes in the d13C value, by feeding an out-expiration air similar to the test subject at the inlet of the sampling point, or by feeding the gas sample at the inlet of the sampling point by flooding the measurement gas with test gas.

## Revendications

1. Procédé pour enregistrer et évaluer des processus métaboliques par une détermination du temps de vidange gastrique ou par une détermination de l'interaction de médicaments lors de la polymédication via une détermination du temps de vidange gastrique à l'aide de l'analyse spécifique des isotopes, lors duquel les temps de vidange gastrique sont déterminés au moyen du quotient de la concentration en ¹³CO₂ et de celle en ¹²CO₂ dans l'air d'expiration du sujet, la valeur du ¹³C dans le gaz respiratoire du sujet étant déterminée par spectroscopie infrarouge non dispersible en continu, en ligne et périodiquement et enregistrée à une période qui est nettement plus courte que la demi-vie biologique de modifications dans le métabolisme ou dans les concentrations dans le pool de sang en circulation et une évaluation cinétique des valeurs de gaz respiratoire étant remplacée ou complétée par une évaluation orientée vers le bilan, en ce qu'une quantité cumulée de ¹³CO₂ qui a été rejetée à un moment déterminé après l'administration d'une substance test est utilisée comme indicateur pour la quantité de substance test qui est libérée par l'estomac jusqu'à ce moment dans l'intestin, la quantité absolue, résolue dans le temps, de CO₂ total rejeté jusqu'à ce moment étant détectée en plus de l'enrichissement en ¹³CO₂ dans le gaz respiratoire pour la détermination des temps de vidange gastrique et la vidange gastrique et l'absorption via l'intestin étant déterminées et détectées séparément à partir des temps de vidange gastrique déterminés et d'autres données d'état détectées, via des modèles de calcul, et des variables indicatrices étant générées pour la vidange gastrique et l'absorption intestinale.

2. Procédé selon la revendication 1, **caractérisé en ce que** la période de la répétition régulière de mesure en ligne est inférieure à 1 minute.

3. Procédé selon la revendication 1 ou 2, **caractérisé en ce que** les valeurs déterminées sont enregistrées et comparées et évaluées par rapport à des valeurs limites prédéfinies.

4. Procédé selon l'une quelconque des revendications précédentes, **caractérisé en ce qu'**on détecte en parallèle la libération de ¹²CO₂ par unité de temps par le patient.

5. Procédé selon l'une quelconque des revendications précédentes, **caractérisé en ce qu'**on détecte en parallèle la vidange gastrique du patient par détermination de valeurs limites prédéfinies.

6. Procédé selon l'une quelconque des revendications précédentes, **caractérisé en ce que**, pour la fixation des valeurs limites du bruit de fond ou des erreurs de mesure pour les mesures indiquées sont prédéfinies et comparées à des valeurs empiriques.

7. Procédé selon l'une quelconque des revendications précédentes, **caractérisé en ce qu'**on détecte également d'autres paramètres supplémentaires pour la description de l'état général, tels que par exemple l'âge, le poids du sujet ainsi que des modèles des gaz respiratoires produits par le corps, comme par exemple les corps cétoniques.

8. Procédé selon l'une quelconque des revendications précédentes, **caractérisé en ce qu'**on établit, à partir des erreurs de mesure estimées, une mesure de fiabilité sous forme d'une plage de dispersion pour les variables indicatrices et **en ce que** des valeurs limites sont prédéfinies pour ces variables indicatrices et leur plage de dispersion, à l'aide desquelles le passage d'une alimentation parentérale à une alimentation entérale est le cas échéant fixé ou généré automatiquement.

9. Procédé selon l'une quelconque des revendications précédentes, **caractérisé en ce qu'**on réalise, au choix, d'abord la variante la plus simple du test de gaz respiratoire, basé uniquement sur l'enrichissement en ¹³CO₂ et **en ce qu'**on fixe une largeur de dispersion maximale pour lesdites variables indicatrices, le test étant répété ou des mesures supplémentaires partielles, telles que la vidange gastrique, étant réalisées lors du dépassement de celle-ci, de manière à n'avoir recours à des modèles d'évaluation plus conséquents et plus fiables que lorsqu'ils sont effectivement nécessaires.

10. Procédé selon l'une quelconque des revendications précédentes, **caractérisé en ce que** la mesure du flux de CO₂ est réalisée via un bypass par un procédé de pression capillaire/différentielle.

11. Procédé selon l'une quelconque des revendications précédentes, **caractérisé en ce que** des mesures erronées de concentration par des éliminations gastriques sont corrigées par une mesure de sel.

12. Procédé selon l'une quelconque des revendications précédentes, **caractérisé en ce que** des tests de plausibilité, en particulier lors de l'apparition de modifications de la valeur d¹³C, peuvent être réalisés par application d'air d'expiration analogue à celui du sujet à l'entrée du prélèvement d'échantillon ou par application de l'échantillon gazeux à l'entrée du prélèvement d'échantillon, en faisant s'écouler un gaz de test avec le gaz de mesure.
